# EUROPEAN PATENT SPECIFICATION

(11) **EP 2 119 420 B1**
(45) Date of publication and mention of the grant of the patent: **04.04.2012**
(21) Application number: 08103763.2
(22) Date of filing: 29.04.2008
(51) Int. Cl.: A61F 13/15

(54) **Process for making an absorbent core with strain resistant core cover**
Verfahren zur Herstellung eines saugfähigen Innenteils mit dehnungsresistentem Innenteilüberzug
Processus de fabrication d'une partie centrale absorbante avec couvercle résistant aux contraintes

(43) Date of publication of application: 18.11.2009
(73) Proprietor: The Procter & Gamble Company, Cincinnati, OH 45202 (US)
(72) Inventor: Schäfer, Jochen, 65824, Schwalbach (DE); Graf, Nicole, 64625, Bensheim (DE); Schmidt, Mattias, 65510, Idstein (DE)
(74) Representative: Heide, Ute

(56) References cited:
- EP-A- 1 621 167
- EP-A- 1 700 586
- US-A1- 2005 096 623
- US-A1- 2006 004 335
- US-A1- 2006 005 919
- US-A1- 2007 197 987

## Description

### FIELD OF THE INVENTION

The present invention relates to a process for making an absorbent core comprising a nonwoven core cover that offers improved performance on holding back fine particulate material after having been exposed to external strain.

The present invention further relates to an absorbent garment comprising a topsheet, or an apertured topsheet, and an absorbent core made by the process of the present invention.

### BACKGROUND OF THE INVENTION

In absorbent garments nonwoven fabrics are commonly used as a core cover to enclose the absorbent core. When used as core cover, the nonwoven fabric has to meet certain requirements. One of these is to contain the absorbent material that commonly comprises superabsorbent polymer material (SAP) which is typically applied as a powder or as fine particulate material. The core cover should be designed to contain this material in dry state prior to use and also in use when the absorbent material may be contacted with bodily fluids.

In recent years effort has been made to decrease the amount of cellulose fibers, such as fluff pulp, used for the so-called "airfelt" in absorbent cores. Decreasing the amount is desirable for reasons of comfort and appearance due to less bulk in the crotch region. Furthermore, absorbent garments with reduced airfelt content occupy less storage space on the shelf, because they are thinner in the dry state prior to use.

The airfelt in conventional absorbent cores partly helps to immobilize the superabsorbent polymer material (SAP) in dry and wet state as the SAP particles are entangled between the airfelt fibers. Therefore, when the content of airfelt is reduced, other SAP-immobilization techniques have to be employed. For example in EP 1 447 066 (Busam et al.) the SAP is adhered to a substrate layer by using thermoplastic adhesive.

US2006005919 (A1) relates to a method of making stretchable absorbent articles. The method may include steps of providing a continuous substrate layer, providing one or more streams of adhesive fibers and superabsorbent particles, shaping the one or more streams of adhesive fibers and superabsorbent particles, depositing the adhesive fibers and superabsorbent particles on the substrate layer to form a plurality of shaped absorbent cores bound to the substrate layer, and separating the absorbent cores from each other.

US 2007197987 relates to a method of making an absorbent composite including a first fabric, a second fabric, and particles positioned between the two fabrics and absorbent articles made from the absorbent composite. The particles may be secured between the two fabric using adhesive, thermal plastic or combinations thereof. The fabrics may be bonded together in a manner that limits particle movement between the fabrics.

EP 1621167 (A2) relates a process for preparing liquid absorbent structures for forming a pattern of particulate material in a composite material comprising a web material and particulate absorbent material. EP 1621167 (A2) further relates to a method for determining the equipment design and process parameter for such a process. In a particular application, the present invention provides a process.

US 2005096623 (A1) relates to absorbent articles comprising a stretchable substrate and an absorbent composite comprising a layer of adhesive composition in contact with the stretchable substrate. A layer of particulate superabsorbent material may be applied to and held by the adhesive composition. The absorbent composite may be secured to the substrate by the adhesive composition.

US 2006004335 (A1) relates to unitary absorbent cores which include a nonwoven layer having a body-facing surface and a garment-facing surface. At least a portion of a first binding agent may be impregnated into that garment-facing surface. Superabsorbent material may be located on the impregnated surface. The unitary absorbent core can be utilized for absorbing viscous fluids.

However, such immobilization techniques often require production processes wherein the nonwoven core covers have to endure relatively high strains compared to processes for producing conventional cores, comprising a comparably high amount of airfelt.

Thus, many absorbent cores that contain a high percentage of SAP still tend to be more likely to show a loss of SAP. Particularly in products featuring an apertured topsheet, SAP lost from the core may get outside of the garment and, when swollen due to the exposure to bodily fluids, stick to the wearers skin (so-called "gel on skin"), which is a phenomenon that should be minimized.

Therefore there is a continuing need to minimize the loss of SAP for absorbent garments with low airfelt-content.

### SUMMARY OF THE INVENTION

The present invention relates to a process for making an absorbent core comprising at least a first nonwoven core cover and a superabsorbent polymer material, the process comprising steps of
a. providing the first nonwoven core cover having an initial air permeability of less then 60 m³/(m²·min)_{;}
b. providing the superabsorbent polymer material;
c. depositing the first nonwoven core cover on a support, the support comprising open areas;
d. depositing the superabsorbent polymer material onto the nonwoven core cover;
wherein the first nonwoven core cover is strained during that process; and wherein the first nonwoven core cover is selected in that it shows an increase in air permeability by less then 18% after the nonwoven core cover having been exposed to a first prescribed strain of 10%, determined according to the test method disclosed herein; and wherein the first nonwoven core cover comprises an amount of at least 80% and at most 95% by weight of spunbond fibers; and
wherein the first nonwoven core cover comprises three layers, wherein one layer of meltblown fibers is sandwiched between two layers of spunbond fibers.

The present invention further relates to an absorbent garment comprising a topsheet, or an apertured topsheet, and an absorbent core made by the process according to the process of the present invention.

### BRIEF DESCRIPTION OF THE DRAWINGS

Figure 1 shows an embodiment of an absorbent garment.
Figures 2, 3 and 4 show embodiments of an absorbent core.
Figures 5 shows an embodiment of the process for making an absorbent core.
Figures 6, 7 and 8 show enlarged views of Figure 5.

### DETAILED DESCRIPTION OF THE INVENTION

### Definitions

As used herein, the following terms have the following meanings:
"Absorbent article" refers to devices that absorb and contain liquid. In one embodiment, the term "absorbent article" refers to devices that are placed against or in proximity to the body of the wearer to absorb and contain the various exudates discharged from the body. Absorbent articles include but are not limited to diapers, adult incontinent briefs, training pants, diaper holders and liners, sanitary napkins and the like.
"Absorbent garment" refers to an absorbent article that is intended to be worn by wearer about the lower torso to absorb and contain the various exudates discharged from the body. Typically, an absorbent garment according to the present invention is disposable.
"Diaper" refers to an absorbent garment generally worn by infants (e.g. babies or toddlers) about the lower torso. Suitable diapers are disclosed in, e.g., U.S. Patent 5,221,274 issued to Buell et al. on June 22, 1993; and U.S. Patent 5,554,145 issued to Roe et al. on September 10, 1996. As used herein the term "diaper" also comprises "pant-like diapers": A pant-like diaper refers to an absorbent garment having fixed sides and leg openings. Pant-like diapers are placed in position on the wearer by inserting the wearer's legs into the leg openings and sliding the pant-like diaper into position about the wearer's lower torso.

Suitable pant-like diapers are disclosed in, e.g., U.S. Patent No. 5,246,433, issued to Hasse, et al. on September 21, 1993.

"Disposable" refers to items that are intended to be discarded after a limited number of uses, frequently a single use (i.e., the original absorbent article as a whole is not intended to be laundered or reused as an absorbent article, although certain materials or portions of the absorbent article may be recycled, reused, or composted). For example, certain disposable absorbent articles may be temporarily restored to substantially full functionality through the use of removable/replaceable components but the article is nevertheless considered to be disposable because the entire article is intended to be discarded after a limited number of uses.

"Comprise," "comprising," and "comprises" is an open ended term that specifies the presence of what follows e.g. a component but does not preclude the presence of other features, elements, steps or components known in the art, or disclosed herein.

"Absorbent core" refers to the region of an absorbent that is intended to absorb and store exudates discharged from the body. Generally, the absorbent core comprises the absorbent material, such as the superabsorbent polymers.

"Core cover" refers to nonwoven fabrics which are intended to at least partly cover or to enclose the absorbent material comprised by the core.

"Nonwoven fabric" refers to a manufactured web of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin. They may be staple or continuous filaments or be formed in situ. The terms "nonwoven fabric" and "nonwoven web" are used interchangeably. The basis weight of nonwoven fabrics is usually expressed in grams per square meter (g/m²) and can be determined according to EDANA method 40.3-90. Generally, nonwoven fabrics may comprise fibers made by nature (natural fibers), made by man (synthetic fibers), or combinations thereof. Example natural fibers include but are not limited to: animal fibers such as wool, silk, fur, and hair; vegetable fibers such as cellulose, cotton, flax, linen, and hemp; and certain naturally occurring mineral fibers.

As used herein "strain" refers to the first substantial elongation of a nonwoven fabric to a length exceeding its initial length, wherein the initial length is the length of a sample of the nonwoven fabric directly after its manufacture. However, nonwovens may experience some minor, generally unintended elongations (not-substantial elongations) after manufacture. For example, the nonwoven fabric is wound up on a roll by the supplier. Not-substantial elongations typically do not extend the nonwoven to more than its initial length plus 2% or even only 1% of its initial length.

Herein "hot melt adhesive" is used according to the definition given in "Adhesion and Adhesives Technology: An Introduction" by Alphonsus V. Pocius (Hanser publishers Munich, 1997). Therein a hot melt is defined as an adhesive applied from the melt and gaining strength upon solidification.

### Absorbent garments

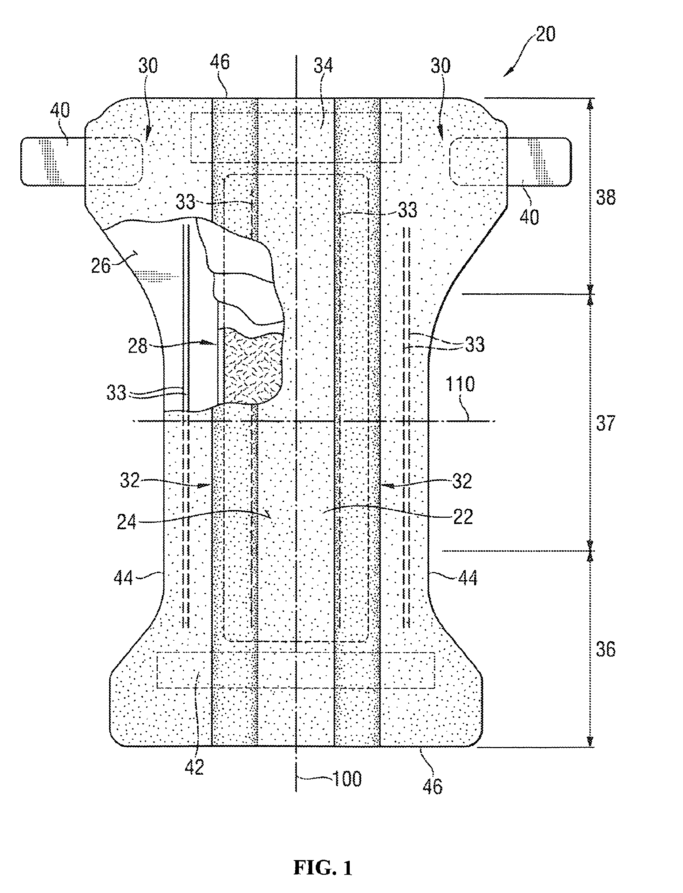

Figure 1 is a plan view of a diaper 20 as an embodiment of an absorbent garment according to the present invention. The diaper is shown in its flat out, uncontracted state (i.e., without elastic induced contraction). Portions of the structure are cut away to more clearly show the underlying structure of the diaper 20. The portion of the diaper 20 that contacts a wearer is facing the viewer. The chassis 22 of the diaper 20 in Figure 1 comprises the main body of the diaper 20. The chassis 22 comprises an outer covering including a liquid pervious topsheet 24 and/or a liquid impervious backsheet 26. The chassis 22 may also include most of or the entire absorbent core 28 encased between the topsheet 24 and the backsheet 26. The chassis 22 may further include side panels 30, leg cuffs 32 with elastic members 33 and a waist feature 34. The leg cuffs 32 and the waist feature 34 typically comprise elastic members. One end portion of the diaper is configured as the front waist region 36 of the diaper 20. The opposite end portion is configured as the rear waist region 38 of the diaper 20. The intermediate portion of the diaper is configured as the crotch region 37, which extends longitudinally between the front and rear waist regions. The crotch region 37 is that portion of the diaper 20 which, when the diaper is worn, is generally positioned between the wearer's legs.

The waist regions 36 and 38 may include a fastening system comprising fastening members 40 preferably attached to the rear waist region 38 and a landing zone 42 attached to the front waist region 36.

The diaper 20 has a longitudinal axis 100 and a transverse axis 110. The periphery of the diaper 20 is defined by the outer edges of the diaper 20 in which the longitudinal edges 44 run generally parallel to the longitudinal axis 100 of the diaper 20 and the end edges 46 run generally parallel to the transverse axis 110 of the diaper 20.

In one embodiment the topsheet of the absorbent garment of the present invention can also be apertured, i.e. the topsheet has a plurality of apertures having an aperture size of at least about 0.2 mm². The topsheet may have an open area of at least about 10%, the open area being the sum of all apertures. The Method to determine the aperture size and open area of the apertured topsheet in context of the present invention is disclosed in EP 0953324.

In certain embodiments at least a part of the topsheet is apertured, for example in such that it is apertured in at least 20%, or 50%, or 80%, or 90%, or 100% of the area overlaying the absorbent core. Due to the apertures the topsheet may not function as a second barrier for the SAP particles. Accordingly, there is an increased need for an absorbent core with improved SAP retaining properties for absorbent garments comprising an apertured topsheet.

The diaper may also include other features as are known in the art including front and rear ear panels, waist cap features, elastics and the like to provide better fit, containment and aesthetic characteristics.

### Absorbent core

The absorbent core has two sides, an upper, body-facing side and a lower, garment-facing side. Furthermore the absorbent core comprises a core cover and absorbent material, comprising at least the SAP.

According to the present invention the core cover described herein may be used at least on one side of the absorbent core to cover the respective side of the absorbent material.

Additionally, the core cover may also be used to cover the body-facing side and the garment-facing side of the absorbent material, in such that the absorbent material is wrapped by the core cover. In these embodiments the absorbent material may either be sandwiched between two separately provided sheets of core cover material, or it may be wrapped by folding one sheet of core cover material, for example in a C-fold, to envelope the absorbent material.

Especially when the nonwoven is intended to cover the body facing side of the absorbent core it may be desirable that it is hydrophilic. In certain embodiments of the present invention the nonwoven may be rendered hydrophilic by means known in the art.

In an alternative embodiment the core cover may be used to cover only the garment-facing side of the absorbent material. However, in certain embodiments it may be preferred that the core cover described below covers at least the body-facing side of the absorbent material.

In embodiments comprising a core cover comprising two separately provided sheets of material, at least one sheet consists of the core cover material of the present invention.

In embodiments comprising a core cover provided by a single sheet of core cover material, the edges of the folded sheet may be sealed together to enclose the absorbent material.

Sealing may be facilitated at least along the longitudinal edges of the absorbent core. Alternatively, the core cover may be sealed completely along all edges.

The amounts of materials used in the absorbent core herein are given in % by weight relative to the basis weight of the whole absorbent core. The whole absorbent core herein includes the core cover.

An absorbent core may comprise any absorbent material that is generally compressible, conformable, non-irritating to the wearer's skin, and capable of absorbing and retaining liquids such as urine and other certain body exudates. An absorbent core may comprise a wide variety of liquid-absorbent materials commonly used in disposable diapers or other absorbent articles. For example soft materials providing a rather fluffy structure with a lot of empty space, such as comminuted wood pulp, creped cellulose wadding, chemically stiffened, modified or cross-linked cellulosic fibers which are generally referred to as "airfelt". However, the absorbent core of the present invention preferably comprises less than 20%, or 15% or 10% or 5% by weight the absorbent core of such an airfelt material. The absorbent core may also be substantially free of, or completely free of airfelt material wherein "substantially free of" means that less than 1% by weight of the absorbent core is airfelt material and "completely free of" means that 0% by weight of the absorbent core is airfelt material.

The absorbent material typically comprises SAP, e. g. in the form of SAP particles, optionally mixed with fibrous materials. The absorbent core as well comprises hot melt adhesive.

The absorbent core may comprise a relatively high amount of SAP of more than 80% or 85% or 90% or 95% by weight of the absorbent core. Furthermore, the absorbent core may comprise a hot melt adhesive, as will be described in more detail below.

According to one embodiment of the present invention the absorbent core comprises the superabsorbent polymer material, the hot melt adhesive and the core cover, wherein the amounts of these materials add up to present 99%, or 100% by weight of the absorbent core.

An absorbent core according to the present invention may for example comprise as a core cover a first nonwoven fabric and a second nonwoven fabric, the SAP may be deposited on the first and second nonwoven fabrics respectively and hot melt adhesive may be deposited in such a way that it at least partly covers or enlaces the deposited SAP on the respective first and second nonwoven fabrics. The absorbent core may then be incorporated in the absorbent garment in such that the first nonwoven fabric faces the topsheet. The first and optionally also the second nonwoven fabric may comprise the core cover of the present invention as will be described below.

It has now been found that production processes for absorbent cores comprising relatively high amounts of SAP of more than 80% or 85% or 90% or 95% by weight of the absorbent core and relatively low amounts of airfelt material of less than 20%, or 15% or 10% or 5% by weight of the absorbent core, and especially absorbent cores that are substantially free or even completely free of airfelt material, often involve steps where the core cover is exposed to higher strain compared to processes used for the production of conventional cores having comparably high amount of airfelt. These strains may cause damages, especially holes in the nonwoven fabric due to the rupture of fibers, and lead to an increased escape of SAP particles through these holes. Therefore the process of the present invention comprises the use of strain resistant nonwoven fabrics that have been found to meet the requirements for enduring the strains occurring during such a process.

Further, an increased loss of SAP may occur when the article is in use. Due to uptake of fluids the SAP swells, tends to expand and may then be hydraulically forced through the core cover. This effect is even more pronounced in cores where the SAP particles are adhered to the core cover by hot melt adhesive, especially if the SAP particles are encapsulated or enlaced by the hot melt adhesive. Due to this encapsulation, the SAP particles may expand by tearing a hole through the core cover, since the expansion in other directions (away from the core cover) is hindered by the hot melt adhesive. This loss may cause the superabsorbent material to stick to a wearer's skin, a phenomenon commonly referred to as "gel on skin".

In view of the above-mentioned reasons core covers should be able to provide sufficient strength and integrity to survive strain intense production processes without substantial damage resulting in holes in the nonwoven and in loss of SAP through these holes. Therefore, a core cover of the present invention should retain the relatively small SAP particles of the superabsorbent polymer material and simultaneously provide a strain resistant fabric that can be effectively employed in fast, strain intense production processes. Furthermore the core cover according to the present invention should withstand the exposure to strain when the absorbent article is in use, for example due to swelling of the superabsorbent polymer material.

### Process for making an absorbent core

The present invention refers to a process for making an absorbent core that comprises at least a first nonwoven core cover and a superabsorbent polymer material (SAP). The process comprises steps of (a) providing the first nonwoven core cover, (b) providing the SAP, (c) depositing the first nonwoven core cover on a support and (d) depositing the SAP on the first nonwoven core cover.

During the process, the first nonwoven core cover is strained. Typically, the strain occurring during such a process strains (elongates) the nonwoven by 10 - 20%, or 12 - 17%, or 12 - 14% of its initial length.

This strain is the first substantial strain the web undergoes after its manufacture. The strain may occur at any time during the production process and may be caused for example by the forces that are applied to hold the core cover on a support with an uneven or apertured surface. Such forces may for example be applied by vacuum means, pulling means (mechanically) or the like. The strain may as well be caused by laying down the SAP at high speed on the first nonwoven core cover. From the further description of the process of the present invention, it is apparent that in certain process executions the nonwoven core cover may locally experience slightly differing strains (for example in embodiments where the nonwoven is strained over a grid). Typically such locally differing strains are applied in a rather regular pattern and thus, for such embodiments, the process strains mentioned above will correspond to an average over the strained area.

It has been found that nonwoven core covers suitable for processes according to the present invention can be characterized by showing an increase in air permeability of less than 18% after having been strained by ε = 10% (ε: prescribed strain, see test methods), or less than 20% after having been strained by ε= 15%. Suitable nonwoven core covers are described in more detail in the section "core cover".

The process may further comprise one or more steps of depositing a hot melt adhesive. The hot melt adhesive may be deposited in the form of fibers such that it enlaces and at least partly immobilizes the SAP. In embodiments where the hot melt adhesive is applied in several steps, in each step a certain portion of the hot melt adhesive is applied. The overall amount of hot melt adhesive given in the section "hot melt adhesive" then corresponds to the sum of all portions of hot melt adhesive applied.

Additionally, the process may comprise steps of providing and depositing a second nonwoven core cover to cover the SAP. The second nonwoven core cover may be deposited in such that the SAP and the optional hot melt adhesive are sandwiched between the first and the second nonwoven core cover.

Alternatively, the process may comprise a step wherein the first nonwoven core cover is folded to wrap the SAP and the optional hot melt adhesive. The first nonwoven core cover may be folded in such that the SAP and the optional hot melt adhesive are enveloped by the first nonwoven.

The steps of the process of the present invention will now be described in more detail.

### (a) Providing the first nonwoven core cover having an initial air permeability of less than 60 m³/(m²·min)

The nonwoven may be taken from a roll where it is wound up, or it may be used directly after its manufacture without intermediate storage.

### (b) Providing the SAP

The SAP may be taken up from a reservoir, for example by a transfer device such as a hopper. The transfer device may have recesses on the surface that can for example determine the amount and distribution pattern of SAP taken up by the transfer device.

### (c) Depositing the first nonwoven core cover on a support, the support comprising open areas.

The support possess an uneven or apertured surface. To provide an uneven surface, the support may comprise a plurality of indents or grooves. A suitable support for example may be a support grid. The support has a first and an opposing second surface. Material, such as the first nonwoven core cover, will be deposited on the first surface. The deposited material may be held on the support by a drawing force, for example by gravitation, an air-stream or by a vacuum which can be applied on the second surface of the support. Thereby, any deposited material, such as the nonwoven core cover and / or the SAP, will be held on the support by suction. Any apertured support on which deposited material can be held by means of passing air through the support herein may also be referred to as vented support.

The support may have the form of a plate, a grid or a belt, for example a rotating drum, a - roll or a transport belt. In embodiments where the support is a drum, the first surface of the support corresponds to the outside surface of the drum and the second surface corresponds to the inside surface of the drum.

Due to the uneven or apertured surface of the support and the drawing force, the nonwoven core cover may be forced into an uneven shape; it may for example bulge corresponding to the apertures, indents or grooves.

### (d) Depositing the SAP on the first nonwoven core cover

The SAP may be moved by the transfer device from the reservoir to the first nonwoven core cover where the SAP may be rapidly deposited on the first nonwoven web. The SAP may be deposited on the nonwoven in such an amount that the content of SAP in the finished absorbent core exceeds 80%, or 85%, or 90%, or 95% by weight of the absorbent core.

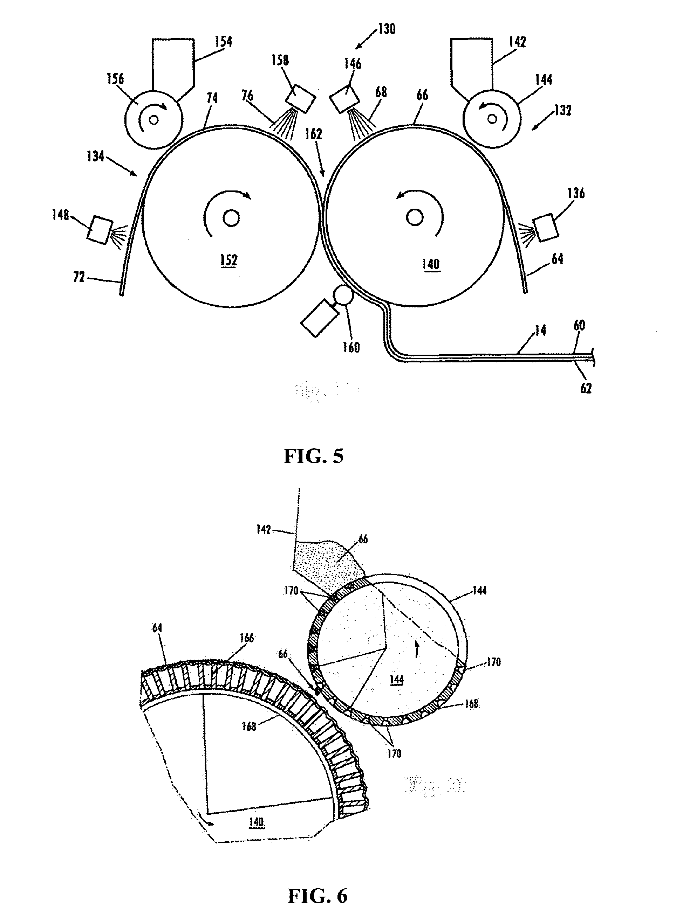

An example for a process of making an absorbent core according to the present invention will be described below with reference to Figures 5 - 8.

Printing system 130 for making an absorbent core 28 in accordance with certain embodiments of this invention is illustrated in Fig. 5 and may generally comprise a first printing unit 132 and a second printing unit 134 for forming the absorbent core 28.

In one embodiment the first printing unit 132 may comprise a first hot melt adhesive applicator (optional) 136 for applying a first portion of hot melt adhesive to the first nonwoven core cover 64, a first rotatable support roll 140 for receiving the first nonwoven core cover 64, a hopper 142 for holding SAP 66, at adhesive printing roll 144 for transferring the SAP 66 to the first nonwoven core cover 64, and a second hot melt adhesive applicator 146 for applying the hot melt adhesive (or a second portion of hot melt adhesive) 68 to the first nonwoven core cover 64 and the SAP 66 material thereon.

The second printing unit 134 may comprise an first hot melt adhesive applicator (optional) 148 for applying a first portion of hot melt adhesive to the second nonwoven core cover 72, a second rotatable support roll 152 for receiving the second nonwoven core cover 72, a second hopper 154 for holding the SAP 74, a second printing roll 156 for transferring the SAP 74 from the hopper 154 to the second nonwoven core cover 72, and a second hot melt adhesive applicator 158 for applying the hot melt adhesive (or a second portion of hot melt adhesive) 76 to the second nonwoven core cover 72 and the SAP 74 thereon.

The printing system 130 may also include a guide roller 160 for guiding the formed absorbent core from a nip 162 between the first and second rotatable support rolls 140 and 152.

The optional first hot melt adhesive applicators 136 and 148 and the second hot melt adhesive applicators 146 and 158 may each be configured as a nozzle system which can provide a relatively thin but wide curtain of hot melt adhesive.

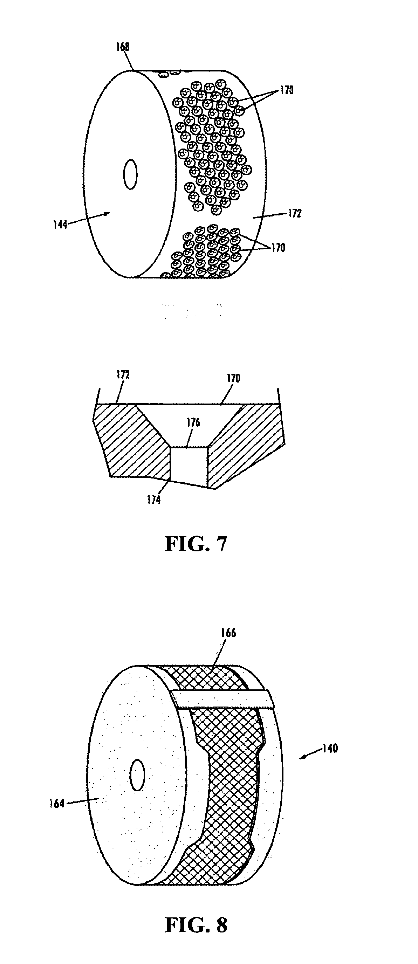

Turning to Fig. 6, portions of the first hopper 142, first support roll 140, and first printing roll 144 are illustrated. As also shown in Fig. 8, the first rotatable support roll 140, which may have the same structure as the second rotatable support roll 152, comprises a rotatable drum 164 and a vented support in form of a grid 166 for receiving the first nonwoven core cover 64.

As also illustrated in Fig. 7, the first printing roll 144, which has the same structure as the second printing roll 156, comprises a rotatable drum 168 and a plurality of SAP indents 170 in a first surface 172 of the drum 168. The indents 170 also illustrated in Fig. 7 may have a variety of shapes, including cylindrical, conical, or any other shape. The indents 170 may lead to an air passage 174 in the drum 168 and comprise a vented cover 176 for holding SAP 66 in the indent and preventing the SAP 66 from falling or being pulled into the air passage 174.

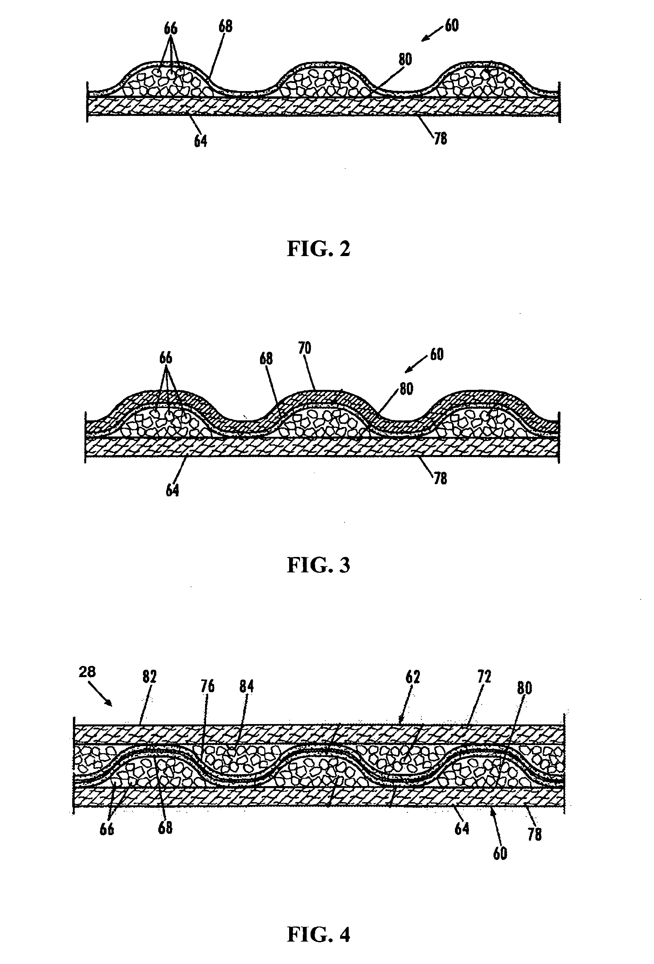

In operation, the printing system 130 receives the first and second nonwoven core covers 64 and 72 into the first and second printing units 132 and 134, respectively, the first nonwoven core cover 64 is drawn by the rotating first support roll 140 past the optional first hot melt adhesive applicator 136 which applies an optional first portion of hot melt adhesive to the first nonwoven core cover 64. A vacuum (not shown) within the first support roll 140 draws the first nonwoven core cover 64 against the support grid 166 and holds the first nonwoven core cover 64 against the first support roll 140. This results in an uneven surface on the first nonwoven core cover 64. The nonwoven core cover 64 will follow the contours of the uneven surface and thereby the nonwoven core cover 64 will assume a ridges and valley shape. The SAP 66 may accumulate in the valleys presented by the first nonwoven core cover 64. The first support roll 140 then carries the first nonwoven core cover 64 past the rotating first printing roll 144 which transfers the SAP 66 from the first hopper 142 to the first nonwoven core cover 64 in a grid pattern. A vacuum (not shown) in the first printing roll 144 may hold the SAP 66 in the indents 170 until the SAP 66 will be delivered to the first nonwoven core cover 64. The vacuum may then be released or air flow through the air passages 174 may be reversed to eject the SAP 66 from the indents and onto the first nonwoven core cover 64. The SAP 66 may accumulate in the valleys presented by the first nonwoven core cover 64. The support roll 140 then carries the printed first nonwoven core cover 64 past the second hot melt adhesive applicator 136 which applies the hot melt adhesive (or a second portion of hot melt adhesive) 68 to cover or to enlace the SAP 66 on the first nonwoven core cover 64. Thereby a first absorbent core precursor 60 is produced. Such an absorbent core precursor is shown in Fig. 2.

In one embodiment of the process according to the present invention the absorbent core precursor 60 will be folded in a C-fold to obtain the absorbent core. Thereby, the first nonwoven core cover envelopes the SAP and the hot melt adhesive. Alternatively, the SAP and the hot melt adhesive are applied only to a part of the surface of the nonwoven core cover. The surface extending beyond the area covered by SAP and hot melt adhesive may then be folded onto the SAP and hot melt adhesive in order to envelope the SAP and hot melt adhesive, thus forming the absorbent core.

In certain embodiments of the process according to the present invention, an additional nonwoven web 70 will be provided and laid down onto the first absorbent core precursor 60 to form an absorbent core. Thereby, the SAP and the hot melt adhesive are sandwiched between the first nonwoven core cover 64 and the additional nonwoven web 70. See for Example Fig. 4. In one embodiment the additional nonwoven web 70 is comprised by the core cover material of the present invention as well.

In another embodiment a second absorbent core precursor 62 may be formed simultaneously with the first absorbent core precursor by the following process steps. The second rotatable support roll draws the second nonwoven core cover 72 past the optional first hot melt adhesive applicator 148 which applies an optional first hot melt adhesive to the second nonwoven core cover 72. The second rotatable support roll 152 then carries the second nonwoven core cover 72 past the second printing roll 156 which transfers the SAP 74 from the second hopper 154 to the second nonwoven core cover 72 and deposits the absorbent polymer material 74 in a grid pattern on the second nonwoven core cover 72 in the same manner as described with regard to the first printing unit 132 above. The second hot melt adhesive applicator 158 then applies the hot melt adhesive (or a second portion of hot melt adhesive) 76 to cover or to enlace the SAP 74 on the second nonwoven core cover 72. The first and second nonwoven core covers 64 and 72 then pass through the nip 162 between the first and second support rolls 140 and 152 for compressing the first absorbent core precursor 60 and second absorbent core precursor 62 together to form the absorbent core 28.

Hence, the uneven surfaces of the vented support grid 166 of the support rolls 140 and 152 respectively determine the distribution of SAP 66 and 74 throughout the absorbent core precursor 60.

### Core cover

The core cover of the present invention is a nonwoven fabric made of synthetic fibers.

Synthetic fibers are man-made fibers, comprising fibers derived from natural sources and mineral sources. Example synthetic fibers, which are derived from natural sources include but are not limited to viscose, polysaccharides (such as starch), rayon and lyocell. Example fibers from mineral sources include but are not limited to polyolefin (such as polypropylene or polyethylene) fibers and polyester fibers. Fibers from mineral sources are derived from petroleum.

Nonwoven webs can be formed by direct extrusion processes during which the fibers and webs are formed at about the same point in time, or by preformed fibers which can be laid into webs at a distinctly subsequent point in time. Example direct extrusion processes include but are not limited to: spunbonding, meltblowing, solvent spinning, electrospinning, and combinations thereof. Nonwoven webs often comprise several layers, which may e.g. be made of different extrusion processes.

As used herein, the term "spunbonded fibers" refers to small diameter fibers, which are formed by extruding molten thermoplastic material as filaments from a plurality of fine, usually circular capillaries of a spinneret. Spunbond fibers are quenched and generally not tacky when they are deposited onto a collecting surface. Spunbond fibers are generally continuous. The spunbond fibers herein may have diameters of from 10µm up to 40µm.

As used herein, the term "meltblown fibers" means fibers formed by extruding a molten thermoplastic material through a plurality of fine, usually circular, die capillaries as molten threads or filaments into converging high velocity gas (e.g. air) streams, which attenuate the filaments of molten thermoplastic material to reduce their diameter. Thereafter, the meltblown fibers are carried by the high velocity gas stream and are deposited on a collecting surface to form a web of randomly dispersed meltblown fibers. The meltblown fibers herein may have diameters of from 0.2µm to less than 10µm.

Example "laying" processes include wet-laying and dry-laying. Example dry-laying processes include but are not limited to air-laying, carding, and combinations thereof typically forming layers. Combinations of the above processes yield nonwovens commonly called hybrids or composites.

The term "nonwoven layer" refers to a layer of fibers that has been extruded by the same technique and have been laid down in a single step. Herein "nonwoven layer of meltblown / spunbond fibers" and "meltblown / spunbond layer" are used interchangeably.

The fibers in a nonwoven web are typically joined to one or more adjacent fibers at some of the overlapping junctions. This includes joining fibers within each layer and joining fibers between layers when there is more than one layer. Fibers can be joined by mechanical entanglement, by chemical bonds, thermal bonds, pressure bonds or by combinations thereof.

While spunbond webs provide relatively good resistance to strain, they offer rather poor area coverage, especially in nonwovens having relatively low basis weights, resulting in pores large enough for the SAP to escape. Furthermore, a spunbond nonwoven having a relatively high basis weight, which may provide better area coverage, may not work well as a core cover because of its relatively higher stiffness and relatively low water permeability. Additionally it may be more difficult to render a spunbond web with high basis weight hydrophilic In embodiments where the body facing side of the absorbent core is covered by the core cover it is desirable that the core cover is water permeable and hydrophilic. Meltblown layers, due to their smaller average pore size, may be suitable to contain even very small particles, but break or rupture more easily when exposed to strain and offer a poor abrasion resistance.

The nonwoven web used for the core cover comprises three or more nonwoven layers each either consisting of spunbond or meltblown fibers. At least two layers consist of spunbond fibers and one or more of meltblown fibers. The nonwoven layers are arranged in that the one or more meltblown layers are sandwiched between the two or more spunbond layers.

The core cover comprises three layers, wherein two layers comprise spunbond fibers (S), one layer comprises melt blown fibers (M) and wherein the meltblown layer is sandwiched between the spunbond layers, forming a configuration known as SMS. Alternatively, the core cover may comprise four layers, wherein two layers may comprise spunbond fibers, two layers may comprises meltblown fibers and wherein the meltblown layers are sandwiched between the spunbond layers, forming a configuration known as SMMS. In another embodiment the core cover may comprise five or more nonwoven layers, wherein two or more nonwoven layers may comprise spunbond fibers and two, or three, or more nonwoven layers may comprise meltblown fibers and wherein the meltblown layers are sandwiched between the spunbond layers, such as SSMMS, SMMMS, SSMMMS or the like.

It has now been found that by adjusting the ratio of spunbond to meltblown fibers in a nonwoven core cover the retention of SAP can be improved even after the nonwoven has been exposed to strain. Therefore, this ratio may be effectively used to adjust the nonwoven to the requirements of strain intense production processes. In such a nonwoven the spunbond fibers act as an efficient scaffold which is able to stabilize the one or more layers of meltblown fibers. The meltblown fibers on the other side provide a fine net which retains the SAP.

The total basis weight of the nonwoven fabric used for the core cover should be high enough to ensure good area coverage and to provide sufficiently small pores. On the other hand the basis weight should not be too high, so that the nonwoven is still compliant and nonirritating to the skin of the wearer. In preferred embodiments, the total basis weight may range from 8 to 20g/m², or 9 to 16g/m², or 10 to 14g/m², for example 13g/m².

The amount of the spunbond nonwoven fibers in a nonwoven fabric consisting of spunbond and meltblown fibers is selected such that the content of spunbond fibers ranges from 80 to 95%, or 82 to 90% of the total basis weight of the nonwoven fabric. It has been found that a rather high content of spunbond fibers increases the strain resistance of the nonwoven fabric and helps to reduce the areas in the meltblown layers that are damaged or ruptured when the web is exposed to strain. It has also been found, that in such a core cover even a relatively low amount of meltblown fibers is sufficient for retaining relatively small particles, even after the nonwoven has been strained.

The nonwoven fabric used for the core cover is further characterized in that it does not show large holes after having been exposed to strain, enabling it to effectively retain the SAP during production of the absorbent article and during use. As characterized by air permeability before and after defined straining determined by the method given in the section TEST METHODS, the nonwoven web of the present invention should show an increase in air permeability of less than 18% after having been strained by ε= 10% (ε: prescribed strain, see test methods), or less than 20% after having been strained by ε= 15%.

The nonwoven fabric used for core cover of the present invention should effectively contain relatively small superabsorbent polymer particles and therefore, it should show an initial air permeability of at most 60 m³/(m²·min), or at most 50 m³/(m²·min), or at most 40 m³/(m²·min)

In certain embodiments the core may be formed by production processes where a vacuum is applied to the nonwoven fabric used for the core cover to hold it on a support and to temporarily immobilize deposited material on the nonwoven. In these embodiments it may be desirable that the nonwoven fabric has an initial air permeability of at least 5 m³/(m²·min), or at least 10 m³/(m²·min), or at least 20 m³/(m²·min).

In certain embodiments the core may be formed by production processes where a vacuum is applied to the nonwoven fabric used for the core cover to hold it on a support and to temporarily immobilize deposited material on the nonwoven. In these embodiments it may be desirable that the nonwoven fabric has an initial air permeability of at least 5 m³/(m²·min), or at least 10 m³/(m²·min), or at least 20 m³/(m²·min).

### Hot melt adhesive

The hot melt adhesive is typically present in a basis weight of 1 - 40 g/m² or 2 - 35 g/m², or 3 - 30 g/m².

Molecular weights herein are given in g/mol unless specified differently.

The hot melt adhesive 68 and 76 may serve to cover and at least partially immobilize the SAP 66 and 74. The hot melt adhesive may at least partially immobilize the SAP by covering or enlacing the SAP. In one embodiment of the present invention, the hot melt adhesive 68 and 76 can be disposed essentially uniformly with the SAP 66 and 74. However, in a certain embodiment, the hot melt adhesive 68 and 76 may be provided as a fibrous layer which is at least partially in contact with the SAP 66 and 74 and partially in contact with the nonwoven core cover 64 and 72.

Figs. 2, 3 and 4 show such a structure, and in that structure, the SAP 66 and 74 is provided as a discontinuous layer on a nonwoven core cover 64 and 72, and a layer of fibrous hot melt adhesive 68 and 76 is laid down onto the layer of SAP 66 and 74, such that the hot melt adhesive 68 and 76 is in direct contact with the SAP 66 and 74, but also in direct contact with a surface 80 and 84 of the nonwoven core cover 64 and 72, in areas where the nonwoven fabric is not covered by the SAP 66 and 74. This imparts an essentially three-dimensional structure to the fibrous layer of hot melt adhesive 68 and 76. In other words, the hot melt adhesive 68 and 76 undulates between the SAP 68 and 76 and the surface of the nonwoven core cover 64 and 72.

Thereby, the hot melt adhesive 68 and 76 may cover the SAP 66 and 74, and thereby immobilizes this material. In a further aspect, the hot melt adhesive 68 and 76 bonds to the nonwoven core cover 64 and 72 and thus affixes the SAP 66 and 74 to the nonwoven core cover 64 and 72. Thus, in accordance with certain embodiments, the hot melt adhesive 68 and 76 immobilizes the SAP 66 and 74 when wet, such that the absorbent core 28 achieves a wet immobilization of more than about 50%, or more than about 60%, 70%, 80% or 90% according to the Wet Immobilization Test described in US Appl. Ser. No. 60/936102. Some hot melt adhesives will also penetrate into the nonwoven core cover 64 and 72, thus providing for further immobilization and affixation.

Of course, while the hot melt adhesives disclosed herein provide a much improved wet immobilization (i.e., immobilization of SAP when the article is wet or at least partially loaded), these hot melt adhesives may also provide a very good immobilization of SAP when the absorbent core 28 is dry.

The hot melt adhesive comprises at least one thermoplastic polymer in combination with other thermoplastic diluents such as tackifying resins, plasticizers and additives such as antioxidants.

In certain embodiments, the thermoplastic polymer typically has a weight average molecular weight (Mw) of more than 10,000 and a glass transition temperature (T_{g}) usually below room temperature (25°C), or of less than 22°C, or less than 18°C, or less than 15°C. In certain embodiments T_{g} may be above 0°C > Tg. In embodiments where the thermoplastic polymer has more than one T_{g} the values given refer to the lowest glass transition temperature. The thermoplastic polymer may also have a softening point, as determined by the ASTM Method D-36-95 "Ring and Ball", in the range between 50 °C and 300 °C. In some embodiments the Mw of the thermoplastic polymer is less than 10000000.

In certain embodiments, typical concentrations of the thermoplastic polymer in a hot melt adhesive are in the range of about 20% to about 40% by weight of the hot melt adhesive.

Exemplary polymers are (styrenic) block copolymers including A-B-A triblock structures, A-B diblock structures and (A-B)n radial block copolymer structures wherein the A blocks are non-elastomeric polymer blocks, typically comprising polystyrene, and the B blocks are unsaturated conjugated diene or (partly) hydrogenated versions of such. The B block is typically isoprene, butadiene, ethylene/butylene (hydrogenated butadiene), ethylene/propylene (hydrogenated isoprene), and mixtures thereof.

In exemplary embodiments, the tackifying resin has typically a Mw below 5,000 and a Tg usually above room temperature (25°C), typical concentrations of the tackifying resin in a hot melt are in the range of about 30% to about 60% by weight of the hot melt adhesive. In certain embodiments the tackifying resin has an Mw of more than 1,000.

The plasticizer has a low Mw of typically less than 1,000 and a Tg below room temperature, with a typical concentration of about 0% to about 15% by weight of the hot melt adhesive. In certain embodiments the plasticizer has an Mw of more than 100.

In certain embodiments, the hot melt adhesive 68 and 76 is present in the form of fibers. In some embodiments, the fibers will have an average thickness of about 1 to about 50 micrometers or about 1 to about 35 micrometers and an average length of about 5 mm to about 50 mm or about 5mm to about 30 mm..

Optionally, a part of the hot melt adhesive, for example an amount of 0 - 10 g/m², may already be deposited on the nonwoven core covers 64 and 72 before application of the SAP 66 and 74 for enhancing adhesion of both the SAP 66 and 74 and the rest of the hot melt adhesive 68 and 76, which is deposited after the SAP has been deposited, to the respective nonwoven core covers 64 and 72.

Said part of the hot melt adhesive may be applied to the nonwoven core covers 64 and 72 by any suitable means, but according to certain embodiments, may be applied in about 0.5 to about 1mm wide slots spaced about 0.5 to about 2 mm apart.

### TEST METHODS

Using a TexTest Instruments Air Permeability Tester FX 3300 LABOTESTER III (available from TexTest Instruments, Schwerzenbach, Switzerland) measure the air permeability of the samples according to EDANA 140.2-99 with the following settings.

Samples are conditioned 24 hours and measured at 23°C, 50% relative humidity. Samples that are intended to be strained are conditioned before the strain is applied. The straining has to be carried out at 23°C, 50% relative humidity as well.

Using a circular test area of 20 cm² and a pressure drop of p = 125 Pa

Report results in m³/(m²·min) as the arithmetic mean of 5 single measurements taken on different samples.

### Straining method and apparatus

The straining is suitably exercised with an apparatus as described in the following. A suitable device shall have two clamps. The two clamps have a longer edge defining their width. The width of the clamps is 200 mm and the clamps are capable of holding the test piece securely across their full width without damage. The clamps shall be oriented in such that their longer edges are parallel and shall be movable in a direction perpendicular to their longer edges. The device shall be capable of extending a test sample at a constant rate of 3cm/sec to a predetermined length (by moving the two clamps away from each other, see below).

The clamps will be suitable to the task of securely holding the sample without damaging it and have a clampdown force enough to hold the sample securely without slippage in the strained mode, and have a smooth surface from which the areas of the sample in contact with the clamps will not be damaged.

The straining procedure shall consist of the following steps:
Cut a web sample to 50 cm length in the intended direction of straining, and 15 cm in the direction perpendicular to the direction of straining;
Secure the sample between the pair of clamps in such that the sample will be strained in machine direction of the nonwoven sample (machine direction being the direction of production of the nonwoven).

Move the second clamp away from the first clamp carefully just until the sample reaches its original full flat-out length, i.e. it should be wrinkle-free and without bows between the clamps, however the sample will not be strained during this step over its original length. Stop the clamps in the position when this state is reached. Measure and record the unstrained length *l₀* as the edge-to-edge distance between the clamps (all lengths are suitably measured with an accuracy of +/- 1mm). The unstrained length *l₀* should be 30 cm.

Stain the sample at a rate of about 3cm/sec until the strained length *l* = *l₀ + Δl* is reached, measured as the edge-to-edge distance between the clamps, where *Δl = l₀ ·* ε/ *100* is the elongation and ε the prescribed strain (expressed in %). Stop the clamps in this position and hold them for between 1 and 3 seconds. Then move the clamps back to a position where the sample is hanging freely between them and not experiencing any strain, and remove the sample.

The air permeability of the strained samples shall be measured immediately after having strained them following the above procedure. The area of the sample submitted to air permeability testing shall be that which has been in the central position of the straining, i.e. at approximately equal distance between the two clamps in the direction of the straining, and between the free edges in the direction perpendicular to the direction of straining.

Unstrained samples shall be measured as obtained, e.g. from a roll. The samples are to be handled with care and no excessive crumpling or other mechanically stressful treatments should be exercised on them prior to measurement.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A process for making an absorbent core comprising at least a first nonwoven core cover and a superabsorbent polymer material, the process comprising steps of
a. providing the first nonwoven core cover having an initial air permeability of less than 60 m³/(m²·min);
b. providing the superabsorbent polymer material;
c. depositing the first nonwoven core cover on a support, the support comprising open areas;
d. depositing the superabsorbent polymer material onto the nonwoven core cover;
wherein the first nonwoven core cover is strained during that process;
and wherein the first nonwoven core cover is selected in that it shows an increase in air permeability by less than 18% after the nonwoven core cover having been exposed to a first prescribed strain of 10% *determined according to the test method disclosed herein; and*
wherein the first nonwoven core cover comprises an amount of at least 80% and at most 95% by weight of spunbond fibers; *and*
wherein the first nonwoven core cover comprises three layers, wherein one layer of meltblown fibers is sandwiched between two layers of spunbond fibers.

2. The process of any of the preceding claims, wherein the absorbent core comprises at least 80% by weight of superabsorbent polymer material.

3. The process of any of the preceding claims, wherein the absorbent core additionally comprises a hot melt adhesive and wherein the process comprises one or more steps of depositing the hot melt adhesive.

4. The process of claim 3 wherein the hot melt adhesive is deposited such that the superabsorbent polymer is enlaced by the hot melt adhesive.

5. The process of any of the preceding claims, wherein the support has two opposite surfaces and wherein the first nonwoven core cover is deposited on the first surface and a vacuum is applied on the second surface of the support.

6. The process of any of the preceding claims further comprising steps of providing a second nonwoven web and of depositing that second nonwoven web onto the absorbent polymer material and the optional hot melt adhesive.

7. The process of any of the preceding claims, wherein the first or second nonwovens, or both have a basis weight in the range of 8 - 20 g/m².

8. The process according to any of the claims 1 - 5, comprising an additional step wherein the first nonwoven core cover is folded to wrap the superabsorbent polymer material and the optional hot melt adhesive.

9. An absorbent garment comprising a topsheet, or an apertured topsheet, and an absorbent core made by the process according to any of the claims 1 - 8.

## Patentansprüche

1. Verfahren zum Herstellen eines Absorptionskerns, der mindestens eine erste Vlieskernabdeckung und ein Superabsorber-Polymermaterial umfasst, wobei das Verfahren die folgenden Schritte umfasst:
a. Bereistellen der ersten Vlieskernabdeckung mit einer anfänglichen Luftpermeabilität von weniger als 60 m³/(m² Min.);
b. Bereitstellen des Superabsorber-Polymermaterials;
c. Aufbringen der ersten Vlieskernabdeckung auf einen Träger, wobei der Träger offene Bereiche umfasst;
d. Aufbringen des Superabsorber-Polymermaterials auf die Vlieskernabdeckung;
wobei die erste Vlieskernabdeckung während dieses Verfahrens beansprucht wird;
und wobei die erste Vlieskernabdeckung so ausgewählt wird, dass sie eine Erhöhung der Luftpermeabilität von weniger als 18 % aufweist, nachdem die Vlieskernabdeckung einer ersten festgelegten Beanspruchung von 10 % ausgesetzt wird, die gemäß dem hierin offenbarten Prüfverfahren bestimmt wird; und
wobei die erste Vlieskernabdeckung Spinnvliesfasern in einer Menge von mindestens 80 Gew.-% bis höchstens 95 Gew.-% umfasst; und
wobei die erste Vlieskernabdeckung drei Schichten umfasst, wobei eine Schicht schmelzgeblasener Fasern zwischen zwei Schichten Spinnvliesfasern beidseitig bedeckt wird.

2. Verfahren nach einem der vorstehenden Ansprüche, wobei der Absorptionskern mindestens 80 Gew.-% Superabsorber-Polymermaterial umfasst.

3. Verfahren nach einem der vorstehenden Ansprüche, wobei der Absorptionskern außerdem einen Heißschmelzkleber umfasst, und wobei das Verfahren einen oder mehere Schritte des Aufbringens des Heißschmelzklebers umfasst.

4. Verfahren nach Anspruch 3, wobei der Heißschmelzkleber derart aufgebracht wird, dass das Superabsorber-Polymer mit dem Heißschmelzkleber durchsetzt wird.

5. Verfahren nach einem der vorherigen Ansprüche, wobei der Träger zwei gegenüberliegende Oberflächen aufweist und wobei die erste Vlieskernabdeckung auf die erste Oberfläche aufgebracht wird und die zweite Oberfläche des Trägers mit einem Vakuum beaufschlagt wird.

6. Verfahren nach einem der vorstehenden Ansprüche, ferner umfassend die Schritte des Bereitstellens einer zweiten Vliesbahn und des Aufbringens dieser zweiten Vliesbahn auf das Absorptionspolymermaterial und den optionalen Heißschmelzkleber.

7. Verfahren nach einem der vorstehenden Ansprüche, wobei der erste oder der zweite Vlies oder beide ein Basisgewicht im Bereich von 8 bis 20 g/m² aufweisen.

8. Verfahren nach einem der Ansprüche 1 bis 5, umfassend einen zusätzlichen Schritt, wobei die erste Vlieskernabdeckung derart gefaltet wird, dass sie das Superabsorber-Polymermaterial und den optionalen Heißschmelzkleber einhüllt.

9. Absorptionskleidungsstück, umfassend eine Oberschicht oder eine mit Öffnungen versehene Oberschicht und einen Absorptionskern, der durch das Verfahren nach einem der Ansprüche 1 bis 8 hergestellt wird.

## Revendications

1. Procédé de fabrication d'une âme absorbante comprenant au moins une première couverture d'âme non tissée et un matériau polymère superabsorbant, le procédé comprenant les étapes consistant à
a. fournir la première couverture d'âme non tissée ayant une perméabilité à l'air initiale inférieure à 60 m³/(m²·min);
b. fournir le matériau polymère superabsorbant ;
c. déposer la première couverture d'âme non tissée sur un support, le support comprenant des zones ouvertes ;
d. déposer le matériau polymère superabsorbant sur la couverture d'âme non tissée ;
dans lequel la première couverture d'âme non tissée est contractée durant ce procédé ;
et dans lequel la première couverture d'âme non tissée est choisie en ce qu'elle présente une augmentation de perméabilité à l'air de moins de 18 % après que la couverture d'âme non tissée a été exposée à une première déformation prescrite de 10 % déterminée selon le procédé de test décrit ici ; et
dans lequel la première couverture d'âme non tissée comprend une quantité d'au moins 80 % et au plus 95 % en poids de fibres filées-liées ; et
dans lequel la première couverture d'âme non tissée comprend trois couches, dans lequel une couche de fibres soufflées en fusion est intercalée entre deux couches de fibres filées-liées.

2. Procédé selon l'une quelconque des revendications précédentes, dans lequel l'âme absorbante comprend au moins 80 % en poids de matériau polymère superabsorbant.

3. Procédé selon l'une quelconque des revendications précédentes, dans lequel l'âme absorbante comprend en outre un adhésif thermofusible et où le procédé comprend une ou plusieurs étapes consistant à déposer l'adhésif thermofusible.

4. Procédé selon la revendication 3, dans lequel l'adhésif thermofusible est déposé de telle sorte que le polymère superabsorbant est entouré par l'adhésif thermofusible.

5. Procédé selon l'une quelconque des revendications précédentes, dans lequel le support a deux surfaces opposées et dans lequel la première couverture d'âme non tissée est déposée sur la première surface et un vide est appliqué sur la deuxième surface du support.

6. Procédé selon l'une quelconque des revendications précédentes, comprenant en outre les étapes de fourniture d'une deuxième nappe non tissée et de dépôt de cette deuxième nappe non tissée sur le matériau polymère absorbant et l'adhésif thermofusible facultatif.

7. Procédé selon l'une quelconque des revendications précédentes, dans lequel les premier ou deuxième non-tissés, ou l'un et l'autre ont une masse surfacique de l'ordre de 8 à 20 g/m².

8. Procédé selon l'une quelconque des revendications 1 à 5, comprenant une étape supplémentaire dans laquelle la première couverture d'âme non tissée est pliée pour envelopper le matériau polymère superabsorbant et l'adhésif thermofusible facultatif.

9. Vêtement absorbant comprenant une feuille de dessus, ou une feuille de dessus perforée, et une âme absorbante fabriquée par le procédé selon l'une quelconque des revendications 1 à 8.
